# EUROPEAN PATENT SPECIFICATION

(11) **EP 3 858 322 B1**
(45) Date of publication and mention of the grant of the patent: **11.11.2026**
(21) Application number: 19866040.9
(22) Date of filing: 22.04.2019
(51) Int. Cl.: A61K 6/62, A61K 6/887, A61K 6/30, A61K 6/71, A61K 6/16, C09D 133/06, A61K 6/66

(54) **DENTAL COMPOSITION**
DENTALE ZUSAMMENSETZUNG
COMPOSITION DENTAIRE

(30) Priority: 26.09.2018 JP 2018179878
(43) Date of publication of application: 04.08.2021
(73) Proprietor: GC Corporation, Sunto-gun, Shizuoka 410-1307 (JP)
(72) Inventor: JIN, Satoshi, Tokyo 174-8585 (JP); KIMURA, Takumasa, Tokyo 174-8585 (JP); HIRANO, Kyousuke, Tokyo 174-8585 (JP)
(74) Representative: Müller-Boré & Partner Patentanwälte PartG mbB
(86) International application number: PCT/JP2019/017020
(87) International publication number: WO 2020/066099

(56) References cited:
- EP-A2- 2 077 103
- JP-A- 2014 015 408
- JP-A- 2014 015 408
- JP-A- 2016 166 138
- JP-A- 2016 532 640
- US-A1- 2006 241 205
- US-A1- 2016 081 885
- US-B2- 6 903 134

## Description

### FIELD OF THE INVENTION

The present invention relates to a dental composition.

### BACKGROUND OF THE INVENTION

In dental treatment, a paste-like photocurable composition commonly referred to as a composite resin is used to repair teeth damaged by caries, fractures, or the like.

It is demanded for the cured resin tooth of a composite resin to have aesthetic appearance similar to that of natural teeth. In addition to a high color matching of each patient's natural teeth with a cured resin tooth, it is also important for the composite resin to have high photostability so that there is minimal color change when the composite resin is exposed to ultraviolet light. In fact, it is required that color changes cannot be readily recognized by the naked eye when a cured resin tooth of a composite resin is exposed to sunlight for 10 hours in accordance with JIS T 6514.

Therefore, a benzotriazole compound is included as an ultraviolet absorber in the composite resin (see, for example, Patent Document 1).

In addition, a phthalate ester fluorescent agent is included in the composite resin (see, for example, Patent Document 1) because natural teeth absorb ultraviolet light and emit weak fluorescence.

In contrast, LED lights emitting a variety of colors have become familiar in recent years due to the development of LEDs. Near-ultraviolet light is less harmful to the human body than ultraviolet light. Therefore, near-ultraviolet LED light has increasingly used in medical practice.

Further, Patent Document 2 describes a self-etching, self-curing dental adhesive which on the one hand is stable on storage as two-component kit and, on the other hand, shows a high shear bond strength on use. Furthermore, Patent Document 3 describes a dental cementation kit for adhering a prosthetic device to tooth structure using a single-part light-curable, self-adhering cement composition, said composition comprising at least one acidic polymerizable monomer having at least one acidic moiety and at least one ethylenically unsaturated group, at least one non-acidic polymerizable monomer having at least one ethylenically unsaturated group, at least one photoinitiator, and at least one filler. Patent Document 4 describes a dental photopolymerizable composition comprising radically polymerizable monomers as well as a first and a second ultraviolet absorbers. Parent Document 5 describes cosmetic or pharmaceutical formulations comprising 2-ethylhexyl 4-(dimethylamino)benzoate as ultraviolet absorber.

### RELATED-ART DOCUMENT

### Patent Documents

Patent Document 1: Japanese Patent Application Laid-Open No. 2014-15408
Patent Document 2: US 2016/081885 A1
Patent Document 3: EP 2 077 103 A2
Patent Document 4: JP2016166138A
Patent Document 5: US6903134B2

### SUMMARY OF THE INVENTION

### Problems to be Solved by the Invention

A phthalate ester-based fluorescent agent absorbs ultraviolet light and near-ultraviolet light and emits fluorescent light. In contrast, a benzotriazole compound absorbs ultraviolet light in which the phthalate ester-based fluorescent agent absorbs, but does not absorb near-ultraviolet light in which the phthalate ester-based fluorescent agent absorbs. As a result, the benzotriazole compound inhibits the absorption of ultraviolet light by the phthalate ester-based fluorescent agent. Thus, even when the components of the composite resin are adjusted, a fluorescence of the cured resin tooth of the composite resin irradiated with either ultraviolet light or near-ultraviolet light to the cured resin tooth of the composite resin cannot be nearly equivalent to that of the natural teeth.

One aspect of the invention is to provide a dental composition that is excellent in photostability of a cured resin tooth and is capable of having a fluorescence of the cured resin tooth of the dental composition nearly equivalent to that of natural teeth upon emission of either ultraviolet light or near-ultraviolet light.

### Means for Solving the Problems

One embodiment of the present invention is defined in the appended claim 1.

### Effects of the Invention

According to one aspect of the present invention, a dental composition that is excellent in photostability of a cured resin tooth and is capable of having a fluorescence of the cured resin tooth of the dental composition nearly equivalent to that of natural teeth upon emission of either ultraviolet light or near-ultraviolet light.

### DETAILED DESCRIPTION OF THE INVENTION

Next, an embodiment for carrying out the present invention will be described.

### [Dental Compositions]

A dental composition of the present embodiment contains a (meth)acrylate, an ultraviolet absorber, and a fluorescent agent.

### [(meth)acrylate]

In the present specification and claims, a (meth)acrylate refers to a compound (e.g., a monomer, an oligomer, a prepolymer, and the like) having a methacryloyloxy group or an acryloyloxy group (hereinafter referred to as (meth)acryloyloxy group).

The (meth)acrylate preferably has two or more (meth)acryloyloxy groups, and particularly preferably has two (meth)acryloyloxy groups.

The (meth)acrylate is preferably free of an acidic group.

Examples of (meth)acrylates free of an acidic group include methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-ethoxyethyl (meth)acrylate, 2-methoxyethyl (meth)acrylate, 2-ethylhexyl (meth)acrylate, benzyl (meth)acrylate, 2-hydroxy-1,3-di(meth)acryloyloxypropane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, trimethylolpropane tri(meth)acrylate, trimethylolethane tri(meth)acrylate, pentaerythritol tri(meth)acrylate, trimethylol methane tri(meth)acrylate, pentaerythritol tetra(meth)acrylate, polybutylene glycol di(meth)acrylate, ethoxylated bisphenol-A di(meth)acrylate, and bisphenol-A diglycidyl(meth)acrylate.

The (meth)acrylate may be used alone or as a combination of two or more kinds.

The content of (meth)acrylate in the dental composition of the present embodiment is preferably from 0.5 to 70% by mass and further preferably from 10 to 60% by mass. When the content of the (meth)acrylate in the dental composition of the present embodiment is 0.5% by mass or more, adhesion of the dental composition improves. When the content of the (meth)acrylate in the dental composition of the present embodiment is 70% by mass or less, the content of other components can be secured, thereby improving the performance of the dental composition.

### [Ultraviolet Absorber]

An ultraviolet absorber has a maximum absorption wavelength in the ultraviolet region, but does not have a maximum absorption wavelength in the range of greater than 320 nm and less than or equal to 500 nm. This makes it difficult to inhibit the absorption of the fluorescent agent even when the cured resin tooth of the dental composition of the present embodiment is irradiated with either ultraviolet light or near-ultraviolet light. As a result, the fluorescence of the cured resin tooth of the dental composition is nearly equivalent to that of natural teeth.

The ultraviolet absorber preferably has a maximum absorption wavelength in the range of greater than 250 nm and less than or equal to 320 nm. This further improves the photostability of the cured resin tooth of the dental composition of the present embodiment.

Further, it is preferred that the ultraviolet absorber does not have a maximum absorption wavelength in the range of greater than 500 nm and less than or equal to 700 nm. Thus, this makes it even more difficult to inhibit the absorption of the fluorescent agent even when the cured resin tooth of the dental composition of the present embodiment is irradiated with either ultraviolet light or near-ultraviolet light. As a result, the fluorescence of the cured resin tooth of the dental composition becomes close to the fluorescence of natural teeth.

The ultraviolet absorber according to the present embodiment is a compound represented by the following general formula, or 2-ethylhexyl 4-(dimethylamino)benzoate, or 4-aminobenzoic acid.

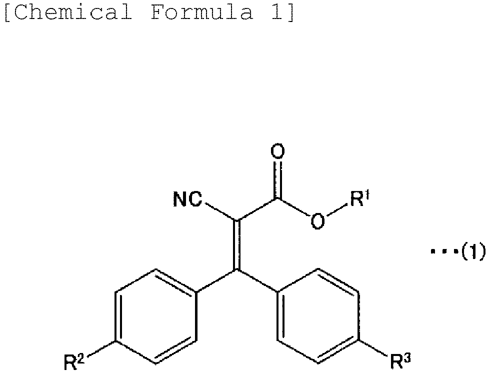

In the formula, R¹ is a linear or branched chain alkyl group having 1 to 30 carbon atoms, and R² and R³ are each independently a hydrogen atom, a linear or branched chain alkyl group having 1 to 30 carbon atoms, or a linear or branched chain alkoxy group having 1 to 30 carbon atoms.

Examples of the alkyl groups in R¹, R², and R³ include a methyl group, ethyl group, 2-ethylhexyl group, and the like.

Examples of the alkoxy group in R² and R³ include a methoxy group, an ethoxy group, a 2-ethylhexyloxy group, and the like.

Examples of the compounds represented by the general formula (1) include a methyl 2-cyano-3,3-diphenylacrylate, ethyl 2-cyano-3,3-diphenylacrylate, 2-ethylhexyl 2-cyano-3,3-diphenylacrylate, and the like.

The ultraviolet absorber may be used alone or as a combination of two or more kinds.

The content of the ultraviolet absorber in the dental composition of the present embodiment is preferably 0.001 to 30% by mass and further preferably 0.1 to 10% by mass with respect to the (meth)acrylate. When the content of the ultraviolet absorber in the dental composition of the present embodiment is 0.001% by mass or more with respect to the (meth)acrylate, the photostability of the cured resin tooth of the dental composition further improves. When the content of the ultraviolet absorber in the dental composition of the present embodiment is 30% by mass or less, the photocurability of the dental composition improves.

### [Fluorescent Agent]

The fluorescent agent is selected from the group consisting of dimethyl 2,5-dihydroxy terephthalate, diethyl 2,5-dihydroxy terephthalate, dimethyl 2,5-diamino terephthalate, diethyl 2,5-diamino terephthalate, and o-phthalaldehyde.

The fluorescent agent may be used alone or as a combination of two or more kinds.

The content of the fluorescent agent in the dental composition of the present embodiment is preferably 0.0001 to 20% by mass and further preferably 0.0005 to 1% by mass with respect to the (meth)acrylate. When the content of the fluorescent agent in the dental composition of the present embodiment is 0.0001% by mass or more and 20% by mass or less with respect to the (meth)acrylate, the fluorescence of the cured resin tooth of the dental composition approaches the fluorescence of natural teeth.

### [Other Components]

The dental composition of the present embodiment may further contain a filler, a photopolymerization initiator, a photopolymerization accelerator, and the like.

### [Fillers]

Examples of the materials constituting the filler include silica, barium glass, fluoroaluminosilicate glass, and the like.

The filler may be surface treated with a silane coupling agent.

Examples of silane coupling agents include 3-glycidyloxytrimethoxysilane, 3-(meth)acryloyloxypropylmethyldimethoxysilane, 3-(meth)acryloyloxypropyltriethoxysilane, 3-(meth)acryloyloxypropylethyldiethoxysilane, 3-(meth)acryloyloxypropylmethyldiethyl diethoxysilane, 2-(meth)acryloyloxyethoxypropyltrimethoxysilane, and the like.

The filler may be used alone or as a combination of two or more kinds.

A median size in a number-based size of the filler is preferably 0.005 to 5 µm and more preferably 0.05 to 0.5 µm.

The content of the filler in the dental composition of the present embodiment is preferably 40 to 95% by mass and further preferably 60 to 80% by mass. When the content of the dental composition of the present embodiment is 40% by mass or more, the mechanical strength of the cured resin tooth of the dental composition improves. When the content of the dental composition of the present embodiment is 95% by mass or less, the working ability of the dental composition improves.

### [Photopolymerization Initiator]

Examples of photopolymerization initiators include camphorquinone, benzyl, diacetyl, benzyl dimethyl ketal, benzyl diethyl ketal, benzyl bis(2-methoxyethyl)ketal, 4,4'-dimethyl(benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10, 10-dioxide, thioxanthone-10-dioxide, benzoin methyl ether, benzoin ethyl ether, isopropyl ether, benzoin isobutyl ether, benzophenone, bis(4-dimethylaminophenyl)ketone, 4,4'-bis(diethylamino)benzophenone, (2,4,6-trimethylbenzoyl)diphenylphosphine oxide (TPO), bis(2,4,6-trimethylbenzoyl)phenylphosphine oxide, and the like.

The photopolymerization initiator may be used alone or as a combination of two or more kinds.

The content of the photopolymerization initiator in the dental composition of the present embodiment is preferably 0.01 to 10% by mass and further preferably 0.1 to 5% by mass with respect to the (meth)acrylate. When the content of the photopolymerization initiator in the dental composition of the present embodiment is 0.01% by mass or more with respect to the (meth)acrylate, the photocurability of the dental composition improves. When the content of the photopolymerization initiator in the dental composition of the present embodiment is 10% by mass or less with respect to the (meth)acrylate, the photostability of the cured resin tooth of the dental composition further improves.

### [Photopolymerization Accelerator]

Examples of photopolymerization accelerators include tertiary amines such as 4-(N,N-dimethylamino)ethylbenzoate, N,N-dimethyl-p-toluidine, triethanolamine, tolyl diethanolamine, 4-dimethylamino methylbenzoate, 4-dimethyl amino isoamylbenzoate; barbituric acid derivatives such as barbituric acid, 1,3-dimethyl barbituric acid, 1,3,5-trimethylbarbituric acid, 1,3,5-triethylbarbituric acid, 5-butylbarbituric acid, 1-benzyl-5-phenylbarbituraic acid, 1-cyclohexyl-5-ethylbarbituric acid, and the like.

The photopolymerization accelerator may be used alone or as a combination of two or more kinds.

The content of the photopolymerization accelerator in the dental composition of the present embodiment is preferably 0.01 to 10% by mass and further preferably 0.1 to 5% by mass with respect to the (meth)acrylate. When the content of the photopolymerization accelerator in the dental composition of the present embodiment is 0.01% by mass or more with respect to the (meth)acrylate, the photocurability of the dental composition improves. When the content of the photopolymerization accelerator in the dental composition of the present embodiment is 10% by mass or less, the photostability of the cured resin tooth of the dental composition further improves.

### [Form and Use of Dental Composition]

The dental compositions of the present embodiment may be provided in the form of, for example, liquids, pastes, and the like.

The dental compositions of the present embodiments include dental composite resins, dental adhesive filler materials, dental adhesives, dental milling resin materials, and the like.

### EXAMPLES

Hereinafter, examples of the present invention will be described, but the present invention is not limited to the described examples.

### [Examples 1 to 9, Comparative Examples 1 to 3]

Bis-GMA (60 parts by mass), 3G (20 parts by mass), and NPG (20 parts by mass) were mixed to obtain a monomer composition.

An ultraviolet absorber (predetermined amount), a fluorescent agent (predetermined amount), a filler (325 parts by mass), a photopolymerization initiator (0.2 parts by mass, and a photopolymerization accelerator (0.5 parts by mass) were added to the monomer composition (100 parts by mass). The mixture was kneaded to be a uniform paste in an agate mortar, and then defoamed under vacuum to obtain a paste-like composite resin.

Table 1 indicates the amount of the ultraviolet absorber and the fluorescent agent that are added.

Details of monomers, ultraviolet absorbers, fluorescent agents, fillers, photopolymerization initiators, and photopolymerization accelerators are as follows.

### Monomers

Bis-GMA: Bisphenol A diglycidyl methacrylate
3G: Triethylene glycol dimethacrylate
NPG: Neopentylglycol dimethacrylate
Ultraviolet absorbers
CDFA: Uvinul3039 (manufactured by BASF) (2-cyano-3,3-diphenylacrylic acid 2-ethylhexyl)
DMABA: 4-(dimethylamino) 2-ethylhexyl benzoate
BT: Tinuvin P (manufactured by BASF) (2-(2-hydroxy-5-methylphenyl)benzotriazole)
Fluorescent agent: Lumilux Blue LZ (manufactured by Honeywell) (diethyl 2,5-dihydroxyterephthalate)
Fillers: Fluoroaluminosilicate glass fillers surface-treated with 0.4 µm median size of 3-glycidyloxytrimethoxysilane in a number-based
Photopolymerization initiator: (±)-camphorquinone
Photopolymerization accelerator: 4-(N,N-dimethylamino) ethylbenzoate (DMABE)

### [Fluorescence of Cured Resin Tooth of Composite Resin]

The composite resin was filled in the shape of central incisor and then exposed to light for 20 seconds using a photopolymerization illuminator G-Light Prima (manufactured by GC) to obtain a cured resin tooth of tooth-shaped composite resin.

The cured resin tooth of composite resin and a natural tooth were placed side by side. The cured resin tooth and the natural tooth were then irradiated by a fluorescent lamp and a near-ultraviolet LED light with ultraviolet light at wavelength of 365 nm and with near-ultraviolet light at 405 nm, respectively. The fluorescence of the cured resin tooth of composite resin was evaluated by observing the comparison of the fluorescence states of the cured resin tooth of composite resin and that of natural tooth. The evaluation criteria for the fluorescence of the cured resin tooth of the composite resin are as follows.

Excellent: The fluorescence state of the cured resin tooth of the composite resin is nearly equivalent to that of the natural tooth.

Good: The fluorescence state of the cured resin tooth of the composite resin is slightly poorer than that of the natural tooth, but there is no problem in use.

Poor: The fluorescence state of the cured resin tooth of the composite resin is inferior to that of the natural tooth, and there is a problem in use.

### [Photostability of Cured Resin Tooth of Composite Resin]

A composite resin was filled in a ring-shaped mold with a diameter of 15 mm and a thickness of 1 mm and then pressed into contact with a polypropylene film. Then, the surface and the back surface of the ring-shaped mold was irradiated with UV light by a photopolymerization illuminator G-light prima (manufactured by GC) at nine locations via a glass plate for 10 seconds to obtain a cured resin tooth of the composite resin (test piece). The test piece was removed from the ring-shaped mold and stored in a dry, dark place at 37°C for 24 hours.

Half of the test piece was covered with aluminum foil, then placed in an optical chamber and immersed in water at 37°C and irradiated with ultraviolet light for 24 hours using a standard xenon white light source with an illumination intensity of 150,000 lux. At this time, the water level was maintained at 10±3 mm above the test piece. Then, the test piece that had been removed from the aluminum foil was stored in a dry dark place at 37°C for 5 days. The color of the non-exposed and exposed portions of the test piece were measured using a colorimeter (manufactured by Minolta Co., Ltd.). The color difference ΔE* was calculated from the following formulae, and the photostability of the test piece was evaluated. $ΔE * = {\left\{{\left(ΔL*\right)}^{2}+{\left(Δa*\right)}^{2}+{\left(Δb*\right)}^{2}\right\}}^{1 / 2}$ $ΔL * = L 1 * - L 2 *$ $Δa * = a 1 * - a 2 *$ $Δb * = b 1 * - b 2 *$

L1* is the lightness index of the non-exposed portion of the test piece, and a1* and b1* are the metric chroma index of the non-exposed portion of the test piece. L2* is the lightness index of the exposed portion of the test piece, and a2* and b2* are the metric chroma index of the exposed portion of the test piece.

**[Table 1]**

| | Amount added [parts by mass] | | | | Fluorescence | | Photostability ΔE |
|---|---|---|---|---|---|---|---|
| | CDFA | DMABA | BT | Fluorescent agent | Ultraviolet light | Near-ultraviolet light | |
| Example 1 | 0.1 | - | - | 0.006 | Excellent | Excellent | 1.96 |
| Example 2 | 0.3 | - | - | 0.006 | Excellent | Excellent | 1.62 |
| Example 3 | 0.5 | - | - | 0.006 | Excellent | Excellent | 1.43 |
| Example 4 | 0.8 | - | - | 0.006 | Excellent | Excellent | 1.33 |
| Example 5 | 1.0 | - | - | 0.006 | Excellent | Excellent | 1.20 |
| Example 6 | 0.3 | - | - | 0.003 | Good | Good | 1.55 |
| Example 7 | 0.3 | - | - | 0.009 | Good | Good | 1.64 |
| Example 8 | 0.3 | - | - | 0.012 | Good | Good | 1.72 |
| Example 9 | - | 0.3 | - | 0.006 | Excellent | Excellent | 1.98 |
| Comparative Example 1 | - | - | 0.1 | 0.028 | Good | Poor | 1.84 |
| Comparative Example 2 | - | - | 0.1 | 0.006 | Poor | Excellent | 1.23 |
| Comparative Example 3 | - | - | 0.3 | 0.083 | Excellent | Poor | 1.19 |

From Table 1, the cured resin teeth of the composite resins of Examples 1 to 9 were irradiated with either an ultraviolet light or a near-ultraviolet light, the fluorescence of the cured resin teeth were nearly equivalent with the fluorescence of the natural tooth or slightly poor than that the natural tooth. Also, the photostability of the cured resin teeth were excellent.

In contrast, the fluorescence of the cured resin tooth of the composite resins in Comparative Examples 1 to 3 resulted in poor fluorescence than that of the natural tooth when either an ultraviolet light and a near-ultraviolet light emitted to the composite resin, because the composite resins contained BT with maximum absorption wavelength at 340 nm as the ultraviolet absorber.

## Claims

1. A dental composition comprising:
a (meth)acrylate;
an ultraviolet absorber; and
a fluorescent agent,
wherein the ultraviolet absorber does not have a maximum absorption wavelength in a range of greater than 320 nm and less than or equal to 500 nm,
wherein the ultraviolet absorber is selected from the group consisting of 2-ethylhexyl 4-(dimethylamino)benzoate, 4-aminobenzoic acid, and a compound represented by the following general formula: 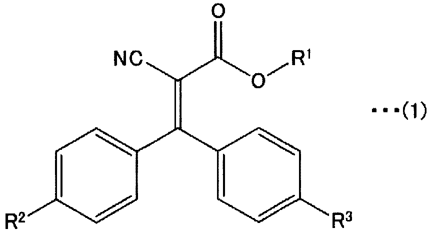 wherein R¹ is a linear or branched chain alkyl group having 1 to 30 carbon atoms, and R² and R³ are each independently a hydrogen atom, a linear or branched chain alkyl group having 1 to 30 carbon atoms, or a linear or branched chain alkoxy group having 1 to 30 carbon atoms, and
wherein the fluorescent agent is selected from the group consisting of dimethyl 2,5-dihydroxy terephthalate, diethyl 2,5-dihydroxy terephthalate, dimethyl 2,5-diamino terephthalate, diethyl 2,5-diamino terephthalate, and o-phthalalde-hyde.

## Patentansprüche

1. Dentalzusammensetzung, umfassend:
ein (Meth)acrylat;
einen Ultraviolettabsorber; und
ein Fluoreszenzmittel,
wobei der Ultraviolettabsorber keine maximale Absorptionswellenlänge in einem Bereich von mehr als 320 nm bis weniger als oder gleich 500 nm aufweist,
wobei der Ultraviolettabsorber aus der Gruppe, bestehend aus 2-Ethylhexyl-4-(dimethylamino)benzoat, 4-Aminobenzoesäure und einer Verbindung, dargestellt durch die folgende allgemeine Formel, ausgewählt ist: 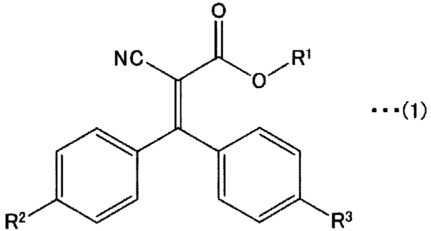 wobei R¹ eine lineare oder verzweigtkettige Alkylgruppe mit 1 bis 30 Kohlenstoffatomen ist und R² und R³ jeweils unabhängig voneinander ein Wasserstoffatom, eine lineare oder verzweigtkettige Alkylgruppe mit 1 bis 30 Kohlenstoffatomen oder eine lineare oder verzweigtkettige Alkoxygruppe mit 1 bis 30 Kohlenstoffatomen sind,und
wobei das Fluoreszenzmittel aus der Gruppe, bestehend aus Dimethyl-2,5-dihydroxyterephthalat, Diethyl-2,5-dihydroxy-terephthalat, Dimethyl-2,5-diaminoterephthalat, Diethyl-2,5-diaminoterephthalat und o-Phthalaldehyd, ausgewählt ist.

## Revendications

1. Composition dentaire comprenant :
un (méth)acrylate ;
un absorbeur d'ultraviolet ; et
un agent fluorescent,
dans laquelle l'absorbeur d'ultraviolet ne présente pas de longueur d'onde d'absorption maximale dans une plage supérieure à 320 nm et inférieure ou égale à 500 nm,
dans laquelle l'absorbeur d'ultraviolet est sélectionné dans le groupe consistant en 4-(diméthylamino)benzoate de 2-éthylhexyle, acide 4-aminobenzoïque, et un composé représenté par la formule générale suivante : 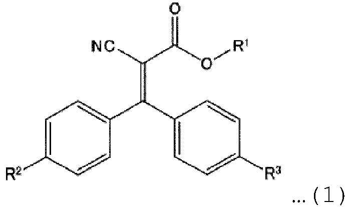
dans laquelle R¹ est un groupe alkyle à chaîne linéaire ou ramifiée présentant 1 à 30 atomes de carbone, et R² et R³ sont chacun indépendamment un atome d'hydrogène, un groupe alkyle à chaîne linéaire ou ramifiée présentant 1 à 30 atomes de carbone, ou un groupe alcoxy à chaîne linéaire ou ramifiée présentant 1 à 30 atomes de carbone, et
dans laquelle l'agent fluorescent est sélectionné dans le groupe consistant en diméthyl 2,5-dihydroxy téréphtalate, diéthyl 2,5-dihydroxy téréphtalate, diméthyl 2,5-diamino téréphtalate, diéthyl 2,5-diamino téréphtalate, et o-phtalaldéhyde.
